# EUROPEAN PATENT APPLICATION

(11) **EP 0 891 771 A1**
(43) Date of publication of application: **20.01.1999**
(21) Application number: 97304994.3
(22) Date of filing: 08.07.1997
(51) Int. Cl.: A61K 31/195, A61K 31/375

(54) **Compositions comprising lysine and ascorbate compounds for the treatment and prevention of cardiovascular diseases**

(71) Applicant: Health Now, Inc., San Francisco, CA 94102 (US)
(72) Inventor: Rath, Matthias, NL-7609 RA Almelo (NL)
(74) Representative: Stoner, Gerard Patrick

(57) **Abstract**

A therapeutic lysine-based composition and methods for its use in the prevention and treatment of cardiovascular disease is disclosed. The composition includes at least one lysine compound. The composition may also preferentially include ascorbate, proline and vitamin D or compounds thereof. The composition may also include N-acetyglucosamine and other compounds restoring and maintaining optimum biological function of the vascular wall. A patient at risk of developing or with a pre-existing cardiovascular disease is treated by administering orally or parenterally a desired dosage of the composition on a daily basis.

## Description

The present invention relates generally to compositions effective in the prevention and treatment of cardiovascular disease and more particularly to compositions based on lysine and/or pharmaceutically acceptable salts of lysine.

### Background

Lipoprotein(a) ("Lp(a)") structurally resembles low density lipoprotein ("LDL") in that both share a lipid apoprotein composition, apolipoprotein B-100 ("apo-B"), the ligand by which LDL binds to LDL receptors present on the interior surfaces of arterial walls. The unique feature of Lp(a) is an additional glycoprotein, designated apoprotein(a) ("apo(a)"), which is linked to apo-B by disulfide groups. The cDNA sequence of apo(a) shows a striking homology to plasminogen, with multiple repeats of kringle 4, one kringle 5, and a protease domain. The isoforms of apo(a) vary in range of 300 to 800 kD and differ mainly in their genetically determined number of kringle 4 structures. Apo(a) has no plasmin-like protease activity. Serine protease activity, however, has been demonstrated. Like plasminogen, Lp(a) has been shown to bind lysine-sepharose, immobilized fibrin and fibrinogen, and the plasminogen receptor on endothelial cells. Furthermore, Lp(a) has been demonstrated to bind to other components of the arterial wall like fibronectin and glycosaminoglycans. The nature of these bindings, however, is poorly understood.

Essentially all human blood contains Lp(a). There can, however, be a thousand-fold range in its plasma concentration between individuals. High levels of Lp(a) are associated with a high incidence of cardiovascular disease. (Armstrong, Dahlem, Miles, Rath, Zenker) The term "cardiovascular disease" is intended to refer to all pathological states leading to a narrowing and/or occlusion of blood vessels throughout the body, but particularly atherosclerosis, thrombosis and other related pathological states, especially as they occur in the arteries of the heart muscle and the brain.

For some time, conventional medical treatment of cardiovascular disease has focused on LDL, the so called "bad cholesterol," and strategies for lowering its concentration in the bloodstream. A great many studies have been published ostensibly linking cardiovascular disease with elevated levels of LDL. As a result, most therapies for the prevention and treatment of cardiovascular disease rely on drugs that reduce serum levels of LDL in the bloodstream. More recent studies have found the beneficial effects of lowering LDL levels to be somewhat equivocal. Thus, the efficacy of these drugs and therapies continues to be a source of major debate within the medical community.

Atherosclerotic deposits are also characterized by the local deposition of calcium inside the artery walls. Vitamin D is essential for optimum metabolism of calcium, including the removal of this mineral from the artery walls in the context of their biological restoration.

We therefore note potential for a drug therapy for preventing or treating cardiovascular diseases by (i) reducing damage to blood vessel walls, (ii) reducing the binding potential of Lp(a) to blood vessel walls and by (iii) improving the metabolism and stability of the vascular wall.

There further exists a need for a treatment that employs compounds that are safe to use with few if any complicating and undesired side effects.

It would be desirable to provide a pharmaceutical composition that is inexpensive, has few or no undesired side effects and is available without a doctor's prescription for the prevention and treatment of cardiovascular disease.

It would also be desirable to provide a method of prevention and treatment of cardiovascular disease that can be both prophylactic or therapeutic, depending upon the progression of disease within a particular patient.

It would also be desirable to provide a method of preventing or treating cardiovascular disease that results in few if any undesired side effects and that is inexpensive to carry out.

According to one aspect of the invention, a lysine-based pharmaceutical composition is provided. The composition preferably includes in addition to a lysine or salt thereof, one or more forms of ascorbic acid.

In an embodiment, the composition further comprises the amino acid proline, or a salt thereof, vitamin D, or a salt thereof, and/or N-acetylglucosamine. The composition preferably comprises a pharmaceutical lysine-based composition having one or more lysine compound(s) selected from the group comprising of lysine, lysine hydrochloride, lysine dihydrochloride, lysine orotate, lysine succinate, and lysine glutamate; one or more ascorbate compound(s) selected from the group consisting of ascorbic acid, pharmaceutically acceptable ascorbate salts and mixtures thereof; and a pharmaceutically acceptable carrier, said composition in an amount effective to prevent and treat cardiovascular disease. Alternatively or additionally, the composition includes one or more lysine compound(s) selected from other lysine salts or synthetic lysine analogues including tranexamic acid or epsilon-aminocaproic acid.

In an alternative embodiment, the composition further includes one or more proline compound(s) selected from the group comprising of proline, proline hydrochloride, proline dihydrochloride, proline orotate, proline succinate, proline glutamate; or another acceptable proline salt. Preferably, the lysine compound, proline compound, and vitamin C compound are present respectively in a ratio between 1:1:1 and 1:1:10. Preferably, the therapeutic dose of the composition has 250 mg of the lysine compound, 250 mg of the proline compound and 1000 mg of the ascorbate compound.

In yet another embodiment , the composition has one or more compounds of Cholecalciferol, 1,25-Dihydroxyvitamin D₃ or another pharmaceutically acceptable form of vitamin D.

In another embodiment, the composition further comprises N-acetylglucosamine or a pharmaceutically acceptable compound thereof.

In an embodiment the pharmaceutical lysine-based composition for prevention of cardiovascular disease is one or more lysine compound(s) selected from the group consisting of lysine, lysine hydrochloride, lysine dihydrochloride, lysine orotate, lysine succinate, and lysine glutamate; one or more lysine compound(s) selected from other lysine salts or synthetic lysine analogues including tranexamic acid, epsilon-aminocaproic acid; one or more ascorbate compound(s) selected from the group consisting of ascorbic acid, pharmaceutically acceptable ascorbate salts and mixtures thereof; one or more proline compound(s) selected from the group consisting of proline, proline hydrochloride, proline dihydrochloride, proline orotate, proline succinate, proline glutamate; or another acceptable proline salt; one or more compounds of Cholecalciferol, 1,25-Dihydroxyvitamin D₃ or another pharmaceutically acceptable form of vitamin D; N-acetylglucosamine or a pharmaceutically acceptable compound thereof; and a pharmaceutically acceptable carrier.

In another embodiment, the pharmaceutical composition for the treatment of cardiovascular disease is one or more lysine compound(s) selected from the group consisting of lysine, lysine hydrochloride, lysine dihydrochloride, lysine orotate, lysine succinate, and lysine glutamate; one or more lysine compound(s) selected from other lysine salts or synthetic lysine analogues including tranexamic acid and epsilon-aminocaproic acid; one or more ascorbate compound(s) selected from the group consisting of ascorbic acid, pharmaceutically acceptable ascorbate salts and mixtures thereof; one or more proline compound(s) selected from the group consisting of proline, proline hydrochloride, proline dihydrochloride, proline orotate, proline succinate, proline glutamate; or another acceptable proline salt; one or more compounds of Cholecalciferol, 1,25-Dihydroxyvitamin D₃ or another pharmaceutically acceptable form of vitamin D;
(a) N-acetylglucosamine or a pharmaceutically acceptable compound thereof; and a pharmaceutically acceptable carrier.

In another aspect of the invention, a method for preventing or treating cardiovascular disease is described, comprising the step of administering to a subject a therapeutically effective amount of the lysine-based composition. The treatment has prophylactic value in that it tends to restore and maintain the metabolic function and stability of the vascular wall and decrease the potential for Lp(a) binding and ultimately plaque accumulation. The treatment has therapeutic value in that it appears to halt and reverse the progress of arterial narrowing by restoring the metabolic function and stability of the vascular wall and promoting the release of Lp(a) already bound.

An embodiment of the method of treating cardiovascular disease is by administering to a patient an effective dosage of one or more lysine compound(s) selected from the group consisting of lysine, lysine hydrochloride, lysine dihydrochloride, lysine orotate, lysine succinate, and lysine glutamate; and/or one or more lysine compound(s) selected from other lysine salts or synthetic lysine analogues including tranexamic acid and epsilon-aminocaproic acid one or more ascorbate compound(s) selected from the group consisting of ascorbic acid, pharmaceutically acceptable ascorbate salts and mixtures thereof; one or more proline compound(s) selected from the group consisting of proline, proline hydrochloride, proline dihydrochloride, proline orotate, proline succinate, proline glutamate; or another acceptable proline salt; one or more compounds of Cholecalciferol, 1,25-Dihydroxyvitamin D₃ or another pharmaceutically acceptable form of vitamin D; N-acetylglucosamine or a pharmaceutically acceptable compound thereof; and a pharmaceutically acceptable carrier, said composition in an amount effective to treat cardiovascular disease.

These and other features and advantages of the invention will become more readily understood upon consideration of the following detailed description.

### Brief Description of the Figures

FIG. 1 is an immunoblot of the plasma of guinea pigs from the test described in Example 1. Increase of Lp(a) in plasma of guinea pigs with a hypoascorbic diet. Immunoblot with anti apo(a) antibodies. Lane 1: human control plasma. Lane 2: guinea pig plasma at start of experiment. Lane 3: guinea pig plasma after 10 days of hypoascorbic diet. Lane 4:guinea pig plasma after 20 days of hypoascorbic diet. (HMW: high molecular weight standard)
FIG. 2A is a picture of an aorta of a guinea pig that received an adequate amount of dietary ascorbate, as described in Example 1. Aortas of guinea pigs receiving an adequate amount of ascorbate (A) and receiving a hypoascorbic diet (B) after three weeks.
FIG. 2B is a picture of an aorta of a guinea pig receiving a hypoascorbic diet over a three week period, as described in Example 1.

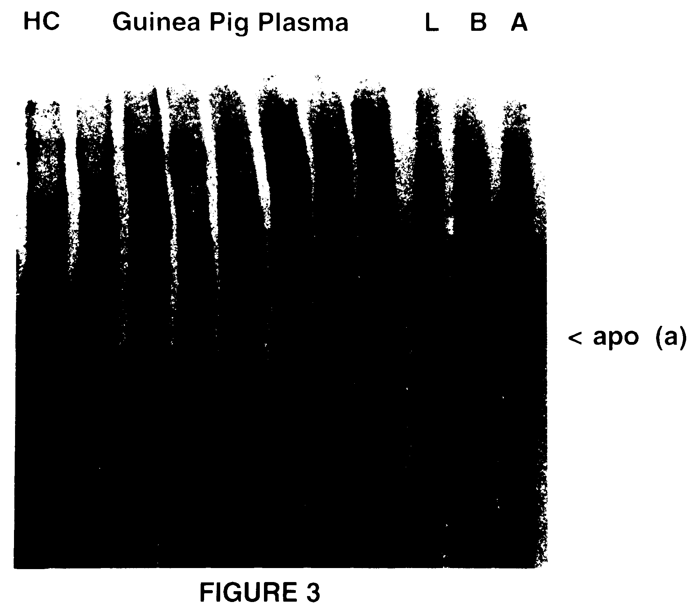

FIG. 3 is an immunoblot of plasma and tissue of Guinea pigs receiving a variety of dietary intake of ascorbate, as described in Example 2. Immunoblot with anti apo(a) antibody. HC: human control plasma, L: liver tissue, B:brain tissue. A:aortic tissue, omogenate of plaque area from figure 2B.

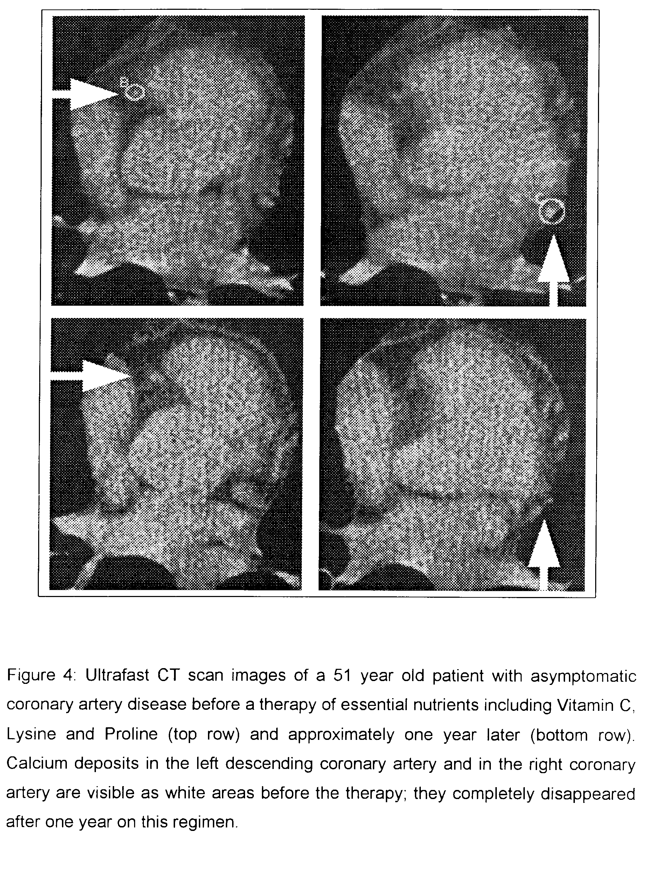

FIG. 4 is an Ultrafast Computed Tomography Scan of a patients' heart before and after this nutritional therapy including, lysine, ascorbate, proline and vitamin D. Ultrafast CT scan images of a 51 year old patient with asymptomatic coronary artery disease before a therapy of essential nutrients including Vitamin C, Lysine, Proline 9top row) and approximately one year later (bottom row). Calcium deposits in the left descending coronary artery an din the right coronary artery are visible as white areas before the therapy; they completely disappeared after one year on this regimen.

### Detailed Description

We have found that animals that have lost the ability to produce ascorbate, such as higher primates and guinea pigs, uniformly produce Lp(a), whereas animals that possess this ability generally do not produce Lp(a). Further, we have found that ascorbate deficiency in humans and guinea pigs tends to raise Lp(a) levels and causes atherosclerosis through deposition of Lp(a) on the inner surface of the arterial wall.

We have also discovered that substances that inhibit binding of Lp(a) to components of the arterial wall, particularly to fibrinogen, fibrin and fibrin degradation products herein identified as binding inhibitors, such as lysine, cause release of Lp(a) from the arterial wall. Thus, ascorbate and such binding inhibitors are not only useful in the prevention of cardiovascular disease, but also for the treatment of such disease. The present invention provides a novel pharmaceutical composition based on lysine that is safe to use e.g without a doctor's prescription. This compound can be used in a method to slow or prevent the onset of cardiovascular disease, as well as slow, stop or even reverse the progress of the disease.

### A. Lysine-Based Composition.

According to one aspect of the present invention, a pharmaceutical composition based on a lysine compound is provided.

In a first embodiment, the composition comprises one or more lysine salts or pharmaceutical derivatives thereof along with a pharmaceutically acceptable carrier.

In a second embodiment, the composition comprises one or more lysine salts or pharmaceutical derivatives thereof along with a combination of an ascorbate compound, along with a pharmaceutically acceptable carrier.

In a third embodiment, the composition of lysine or lysine salts with an ascorbate compound and the amino acid proline or an acceptable salt thereof along with a pharmaceutically acceptable carrier.

In another embodiment, one or more of the first, second or third embodiments are combined with either a Vitamin D compound or N-acetylglucosamine, or a combination thereof along with a pharmaceutically acceptable carrier. Vitamin D is preferentially Cholecalciferol or an acceptable salt thereof.

In all of the above compounds the ascorbate compound may be ascorbic acid, a pharmaceutically acceptable form of an ascorbate salt or a mixture thereof. The lysine compound may be lysine in its electrically neutral form or a pharmaceutically acceptable salt of lysine. Some acceptable salt forms of lysine include lysine hydrochloride, lysine dihydrochloride, lysine succinate, lysine glutamate, and lysine orotate. The proline compound may be proline in its electrically neutral form or a pharmaceutically acceptable salt of proline. Some acceptable salt forms of proline include proline hydrochloride, proline dihydrochloride, proline succinate, proline glutamate, and proline orotate.

The relative percentages of each class of compounds in the compositions may be varied to some degree. It will be understood that each class of compound may be present exclusively in one chemical form, or may be present as a mixture of chemical forms as set forth and described above.

In other embodiments of the composition of the invention, other essential nutrients, that is minerals, trace elements or amino acids, may be added.

It will also be appreciated that the composition just described may consist of a simple mixture of the individual compounds described, or they may be covalently linked or present as ionically bound salts of one another. For example, ascorbate may be covalently linked to lysine.

The constituents described above are generally mixed with a pharmaceutically acceptable carrier, to form tablets or caplets for oral administration. The carrier may contain a binder such as tragacanth, corn starch or gelatin; a disintegrating agent, such as alginic acid, and/or a lubricant such as magnesium stearate. If administration in liquid form is desired, use of sweetening and/or flavoring agents may be used. It will be understood that the composition of the invention may also be delivered parenterally by injection or IV, wherein the carrier may be an isotonic saline solution, a phosphate buffered solution or similar carrier.

### B. Methods of Use.

The composition described above may be used as a prophylactic agent to halt or delay the onset of cardiovascular disease or may be employed to treat an existing cardiovascular condition.

In the case of preventative treatment, it is preferred to administer a composition containing lysine and ascorbate to block any initial Lp(a) binding with the arterial wall, as well as to prevent damage to the vessel wall resulting from a degeneration of the extracellular matrix.

Table I sets forth preferred ranges of dosages of the individual components of a preferred composition. The protocol for prophylatic treatment of patients at risk for cardiovascular disease calls for administration of the doses listed in Table I on a daily basis. Because ascorbate is so quickly cleared from the system, and because it can be irritating to the intestinal lining in high doses until tolerance is reached, it may be preferable to divide the preferred dose into two to four smaller doses that can be administered with meals.

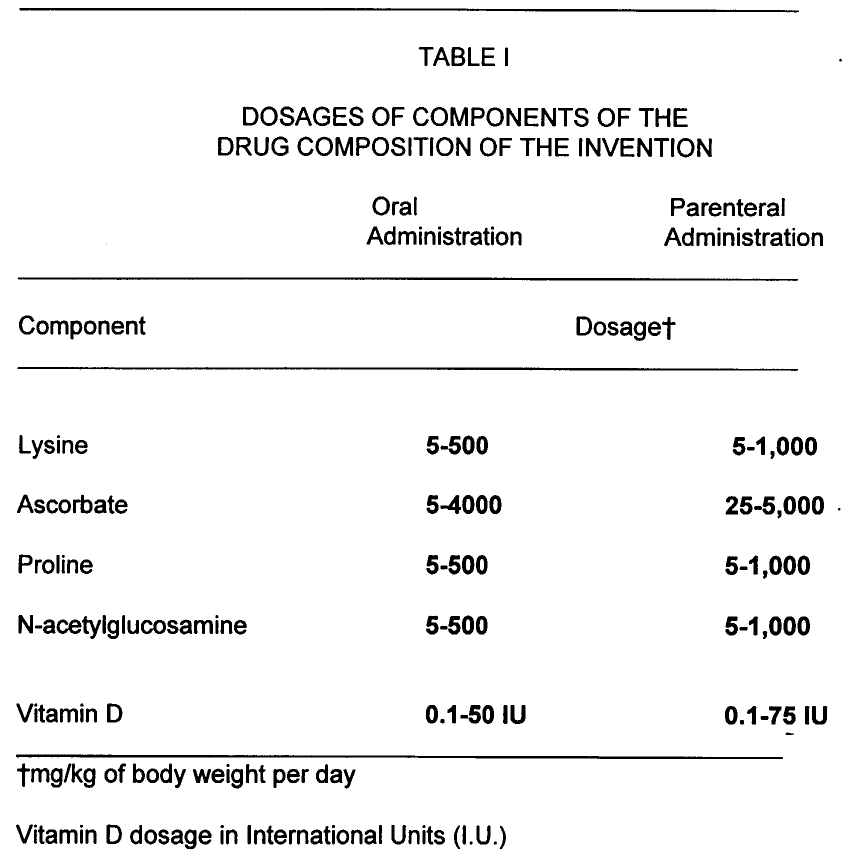

In the treatment of an existing condition of cardiovascular disease, it will be understood that the therapeutic composition described above should include at least one or more lysine compounds and ascorbate. A proline compound and or a vitamin D compound may be added. Suitable dosages are listed in Table I. It should be noted that the concentrations of the individual constituents vary, depending on whether administration is oral or parenteral, and depending on the severity of the disease. It will be appreciated therefore that a subject diagnosed with advanced stages of atherosclerosis should receive a dosage at the higher end of the ranges set forth in Table I.

We find that the present compositions are useful in the treatment of diseases arising form a degeneration of the extracellular matrix, particularly metastasis of cancer. While not wishing to be bound by a particular theory of operation, it appears that lysine is available to inhibit both plasminogen and plasmin, which eventually stimulates the production of collagenase and, ultimately, the collagen network in the extracellular matrix. Such degradation also exacerbates advanced cases of atherosclerosis. Thus, lysine is effective not only to block potential Lp(a) binding sites, but also to inhibit the action of certain proteolytic enzymes that may ultimately result in damage to arterial walls. Thus, in another embodiment of the present invention, natural inhibitors of proteolytic enzymes, such as are found in soybeans, may be added to the composition.

### Experimental

Having disclosed the preferred embodiment of the invention, the following examples are provided by way of illustration only and are not intended to limit the invention.

Guinea pigs are similar to man in their inability to synthesize ascorbate and in their ability to synthesize Lp(a). Guinea pigs are therefore selected as a suitable test animal to test the compositions and methods of the present invention.

### Example 1A: Guinea Pig Study Model

### Development of Atherosclerosis in Female Guinea Pigs

Three female Hartley guinea pigs with an average weight of 800 gm and an approximate age of 1 year were selected for study. One animal received an extreme hypoascorbic diet with approximately 1 mg ascorbate per kg body weight per day. One of the other animals received a diet containing 4 mg ascorbate per kg body weight per day. The remaining animal served as a control and received 40 mg ascorbate per kg body weight per day.

Once after ten days and then again after three weeks, blood was drawn by ear puncture from the anaesthetized animals and collected into EDTA containing tubes. Subsequent to drawing blood at the three weeks, the animals were sacrificed. Plasma was stored at
-80°C until analysis could be conducted. Lp(a) was detected in the plasma by use of an SDS-polyacrylamide gel according to the techniques of Neville (J. Biol. Chem. 257: 13150-13156, 1982), the contents of which are incorporated herein by reference. Forty µL of plasma and 20 mg of arterial wall homogenate were applied in delipidated form per lane of the gel.

Lp(a) presence in the gel was detected by immunological assay using polyclonal anti-human apo(a) antibody (available from Immuno, Vienna, Austria) followed by a rabbit anti-sheep antibody with subsequent silver enhancement (available from Bio-Rad). The determinations of cholesterol and triglycerides were conducted at California Veterinary Diagnostics (Sacramento) using the enzyme assay of Boehringer Mannheim. Plasma ascorbate concentration was determined by the dinitrophenylhydrazine method of Shaffer et al. (J. Biol. Chem. 212: 59, 1955).

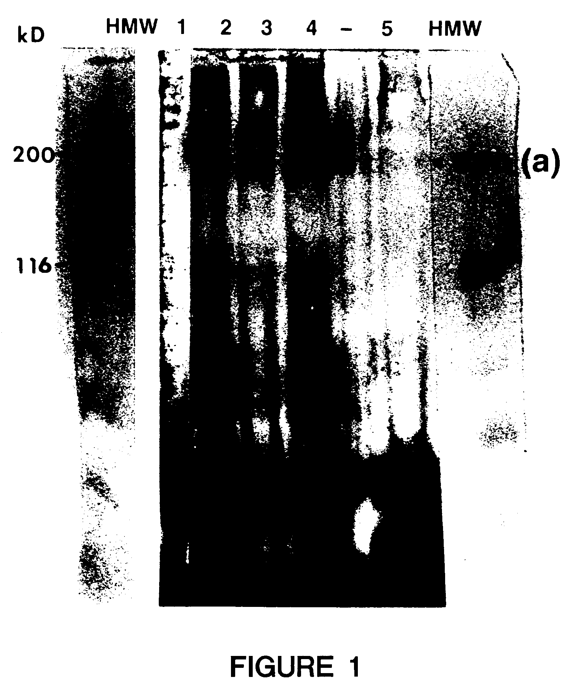

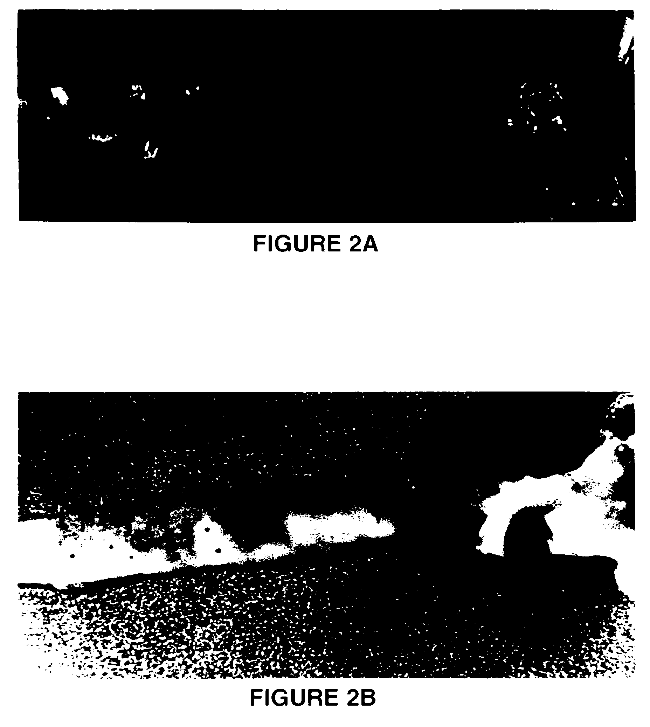

Vitamin C deficiency in the diet led to an increase of Lp(a) in the plasma of the guinea pig, as indicated by a clear band in the immunoblot of the plasma after 10 and 20 days on a hypoascorbic diet (see Figure 1). At necropsy the animals were anaesthetized with Metophane and exsanguinated. Aorta, heart and various other organs were taken for biochemical and histological analysis. The aorta was excised, the adventitial fat was carefully removed, and the vessel was opened longitudinally. Subsequently the aorta was placed on a dark metric paper and a color slide was taken. The picture was projected and thereby magnified by an approximate factor of 10. The circumference of the ascending aorta, the aortic arch and thoracic aorta as well as the atherosclerotic lesions in this area were marked and measured with a digitalized planimetry system. The degree of atherosclerosis was expressed by the ratio of plaque area in relation to the total aortic area defined. The difference in the 3 one-year old animals of the experiment was significant and pronounced lesions were observed in the ascending aorta and the arch of the ascorbate deficient animal (see Figure 2b).

### Example 1B: Guinea Pig Study Model

### Development of Atherosclerosis in Male Guinea Pigs

33 male guinea pigs with a mean weight of 550 gm and an approximate age of 5 months were selected. One group of 8 animals served as a control and received 40 mg ascorbate per kg of body weight per day ("Group A"). To induce hypoascorbemia, 16 animals were fed a diet containing 2 mg of ascorbate per kg body weight per day ("Group B"). Group A and half of Group B were sacrificed after five weeks as described above. The remaining half of Group B was kept alive for 2 more weeks, receiving daily intraperitoneal injection of 1.3 gms of sodium ascorbate per kg of body weight per day. After this period, these animals were sacrificed.

Plasma ascorbate levels were negatively correlated with the degree of atherosclerotic lesion. Total cholesterol levels increased significantly during periods of ascorbate deficiency (see Table II).

The aortas of the guinea pigs receiving a sufficient amount of ascorbate were essentially plaque free, with minimal thickening of the intima in the ascending region. In contrast, the ascorbate-deficient animals exhibited fatty streak-like lesions, covering most parts of the ascending aorta and the aortic arch. In most cases the branching regions of the intercostal arteries of the aorta exhibited similar lipid deposits. The difference in the percentage of lesion area between the control animals and the hypoascorbic diet animals was 25% deposition of lipids and lipoproteins in the arterial wall.

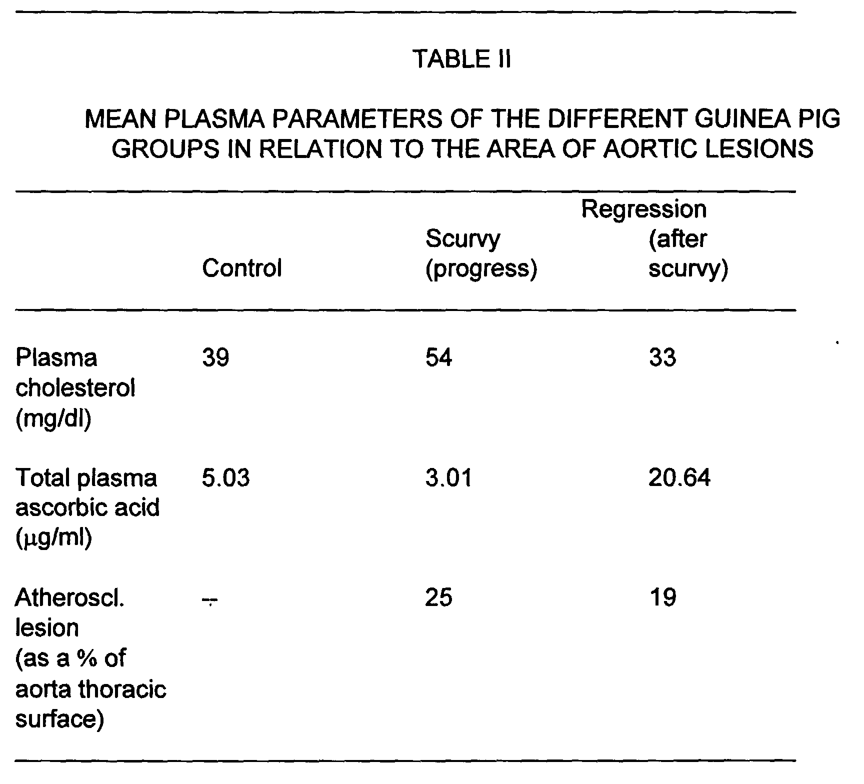

### Example 2

### Effect of Lysine Composition on Disease

Human arterial wall tissue is obtained post mortem from the aorta ascendens of a patient suffering from cardiovascular disease showing an atherosclerotic lesion as evidenced by homogenous intimal thickening. The arterial wall is cut into pieces, with about 100 mg of the cut up tissue homogenized in a glass potter for 1 minute in 2.5 ml of an isotonic solution containing ascorbate alone, ascorbate combined with lysine. The arterial wall segments are then placed in the solution and allowed to incubate. The solution is then decanted off and the concentration of Lp(a) measured according to the techniques discussed above.

It will now be apparent that the measures proposed herein have new and unexpected effectiveness in the prevention and treatment of cardiovascular disease. It should be noted that while certain specific embodiments have been described, changes and modifications made be made in view of the general teachings herein.

### References

Armstrong et al. 1986. Atherosclerosis 62: 249-257.
Berg. K. 1963. Acta Pathologica 59: 369-382..
Dahlem et al. 1986. Circulation 74: 758-765.
Eaton et al. 1987. PNAS USA 84: 3224-3228.
Gonzales-Gronow et al. 1989. Biochemistry 28: 2374-2377.
Hajjar et al. 1989. Nature 339: 303-305.
Harpel et al. 1989. PNAS USA 86: 3847-3851.
McLean et al. 1987. Nature 300: 132-137.
Miles et al. 1989. Nature 339: 301-302.
Rath et al. 1989. Arteriosclerosis 9: 579-592.
Salonen et al. 1989. EMBO Journal 8: 4035-4040.
Zenker et al. 1986. Stroke 17: 942-945.

## Claims

1. A Pharmaceutical lysine-based composition comprising of:
(a) one or more lysine compound(s) selected from the group comprising of lysine, lysine hydrochloride, lysine dihydrochloride, lysine orotate, lysine succinate, and lysine glutamate;
(b) one or more ascorbate compound(s) selected from the group consisting of ascorbic acid, pharmaceutically acceptable ascorbate salts and mixtures thereof; and
(c) a pharmaceutically acceptable carrier, said composition in an amount effective to prevent and treat cardiovascular disease.

2. The composition of claim 1 wherein one or more lysine compound(s) is further selected from other lysine salts or synthetic lysine analogues including tranexamic acid or epsilon-aminocaproic acid.

3. The composition of claim 1 or 2 further comprising one or more proline compound(s) selected from the group comprising of proline, proline hydrochloride, proline dihydrochloride, proline orotate, proline succinate, proline glutamate; or another acceptable proline salt.

4. A composition of any preceding claim comprising one or more compounds of Cholecalciferol, 1,25-Dihydroxyvitamin D₃ or another pharmaceutically acceptable form of vitamin D.

5. A composition of any preceding claim comprising N-acetylglucosamine or a pharmaceutically acceptable compound thereof.

6. The composition of claim 3 wherein the lysine compound, proline compound, and vitamin C compound are present respectively in a ratio between 1:1:1 and 1:1:10.

7. A therapeutic dose of the composition of claim 2 consisting essentially of 250 mg of the lysine compound, 250 mg of the proline compound and 1000 mg of the ascorbate compound.

8. A Pharmaceutical lysine-based composition for the prevention of cardiovascular disease consisting essentially of:
(a) one or more lysine compound(s) selected from the group consisting of lysine, lysine hydrochloride, lysine dihydrochloride, lysine orotate, lysine succinate, and lysine glutamate;
(b) one or more lysine compound(s) selected from other lysine salts or synthetic lysine analogues including tranexamic acid, epsilon-aminocaproic acid;
(c) one or more ascorbate compound(s) selected from the group consisting of ascorbic acid, pharmaceutically acceptable ascorbate salts and mixtures thereof;
(d) one or more proline compound(s) selected from the group consisting of proline, proline hydrochloride, proline dihydrochloride, proline orotate, proline succinate, proline glutamate; or another acceptable proline salt;
(e) one or more compounds of Cholecalciferol, 1,25-Dihydroxyvitamin D₃ or another pharmaceutically acceptable form of vitamin D;
(f) N-acetylglucosamine or a pharmaceutically acceptable compound thereof; and
(g) a pharmaceutically acceptable carrier.

9. A Pharmaceutical lysine-based composition for the treatment of cardiovascular disease consisting essentially of:
(a) one or more lysine compound(s) selected from the group consisting of lysine, lysine hydrochloride, lysine dihydrochloride, lysine orotate, lysine succinate, and lysine glutamate;
(b) one or more lysine compound(s) selected from other lysine salts or synthetic lysine analogues including tranexamic acid and epsilon-aminocaproic acid;
(c) one or more ascorbate compound(s) selected from the group consisting of ascorbic acid, pharmaceutically acceptable ascorbate salts and mixtures thereof;
(d) one or more proline compound(s) selected from the group consisting of proline, proline hydrochloride, proline dihydrochloride, proline orotate, proline succinate, proline glutamate; or another acceptable proline salt;
(e) one or more compounds of Cholecalciferol, 1,25-Dihydroxyvitamin D₃ or another pharmaceutically acceptable form of vitamin D;
(f) N-acetylglucosamine or a pharmaceutically acceptable compound thereof; and
(g) a pharmaceutically acceptable carrier.

10. The use of lysine or pharmaceutically acceptable salt thereof in the manufacture of a medicament for the treatment or prevention of cardiovascular disease, optionally in combination with one or more of
- ascorbic acid and/or pharmaceutically-acceptable ascorbate;
- proline and/or pharmaceutically-acceptable proline salt;
- a pharmaceutically-acceptable form of vitamin B;
- N-acetylglucosamine or pharmaceutically-acceptable derivative thereof;
- carrier.
